# EUROPEAN PATENT APPLICATION

(11) **EP 3 744 243 A1**
(43) Date of publication of application: **02.12.2020**
(21) Application number: 20176780.3
(22) Date of filing: 27.05.2020
(51) Int. Cl.: A61B 5/0478, A61B 5/00, A61B 5/06

(54) **BRAIN SIGNAL TRACKING**

(30) Priority: 28.05.2019 US 201962853409 P; 24.04.2020 US 202016857350
(71) Applicant: Biosense Webster (Israel) Ltd, Yokneam, 2066717 (IL)
(72) Inventor: GOVARI, Assaf, 2066717 Yokneam (IL)
(74) Representative: Small, Gary James

(57) **Abstract**

In one embodiment, a brain signal tracking system includes a probe to be inserted into brain blood vessels, and including an electrode, a signal generator to generate a calibration signal to be emitted by the electrode at different locations in the blood vessels, a tracking subsystem to track locations of the electrode, head surface electrodes to be applied to a head, head surface electrodes configured to detect the calibration signal at respective amplitude values, with a distribution of the respective amplitude values over the head surface electrodes being indicative of a corresponding one of the tracked locations from which the calibration signal was emitted by the electrode, and processing circuitry to find a function to compute a source location in the brain of a brain signal responsively to the distribution of the respective amplitude values of the calibration signal over the head surface electrodes for ones of the tracked locations.

## Description

### RELATED APPLICATION INFORMATION

The present application claims benefit of US Provisional Patent Application No. 62/853,409 of Govari, filed on May 28, 2019, which is hereby incorporated by reference as fully set forth within.

### FIELD OF THE INVENTION

The present invention relates to medical systems, and particularly to cerebrovascular systems.

### BACKGROUND

Electroencephalography (EEG) monitors and records electrical activity of the brain. An EEG generally includes applying electrodes to the outside of the head to record the electrical activity. In some applications electrodes may be applied under the scalp to the surface of the brain.

EEG data is highly sensitive to the placement of the electrodes in their proper positions and also adequate contact between the electrodes and the scalp. The accuracy of electrode placement is highly dependent on the skills of the medical professional administering the EEG. Additionally, correct diagnosis of brain conditions based on EEG data is highly dependent on the accuracy of the EEG data.

US Patent Publication 2004/0049121 of Yaron describes an apparatus for use in a brain of a subject, including an instrument, adapted to be inserted into the brain. A set of one or more electrodes, coupled to the instrument, are adapted to sense electrical activity of the brain and to transmit an electrical activity signal responsive thereto. A location sensor, adapted to be coupled to the instrument transmits a location signal indicative of a location of the instrument. A control unit, analyzes the electrical activity signal and the location signal. The control unit determines, responsive to the analysis, a position of the instrument with respect to an image of the brain, and electrophysiological information regarding tissue at the position.

US Patent Publication 2010/0312096 of Guttman, et al., describes MRI guided cardiac interventional systems configured to generate dynamic (interactive) visualizations of patient anatomy and medical devices during an MRI-guided procedure and may also include at least one user selectable 3-D volumetric (tissue characterization) map of target anatomy, e.g., a defined portion of the heart.

US Patent Publication 2004/0215162 of Putz describes a catheter assembly for intracranial treatment of a patient is disclosed. The assembly comprises an outer catheter having a proximal opening, at least one aperture, a lumen connecting the opening and the at least one aperture and at least one element; and an inner catheter adapted to be received within the lumen and including a passageway and at least one port for transferring fluids between the inner catheter and a tissue region within the patient's brain. The element is preferably a contact for monitoring or stimulating brain tissue or a location marker for allowing identification of the location of the outer catheter within the brain, or both. The assembly facilitates regular accurate placement of the drug delivery catheter at the tissue region without additional extended brain contact during insertion. Drugs can be administered from the drug delivery catheter through ports near the distal end of the depth electrode or fluid can be removed from the brain through the ports.

US Patent Publication 2013/0030408 of Piferi, et al., describes a cannula for transferring a substance to and/or from a patient and includes a tubular support sleeve and a transfer tube. The support sleeve includes a rigid tubular member defining a lumen extending from a proximal end to a distal end of the tubular member. The transfer tube is positioned in the lumen and extends beyond each of the proximal end and the distal end of the tubular member. The tubular member includes a rigid, MRI-compatible material.

US Patent Publication 2003/0045870 of Madsen describes a bipolar coagulator which can be passed through the internal lumen of a ventricular catheter previously implanted into a cranial ventricle of a living subject and engaged in-situ. The bipolar coagulator will provide bipolar electrical arc currents for coagulation cauterization of adherent brain tissues, such as the choroid plexus, which occludes fluid flow into the intake drainage holes in the implanted ventricular catheter and often becomes adherent to the catheter in-situ. The cautery current provided by the bipolar coagulator is direction oriented and spatially controlled; thereby providing a better distribution of electrical current and heat within the surrounding cranial tissues; and thereby avoiding major complications of damage to intracranial structures such as blood vessels as well as avoiding the severe subarachnoid hemorrhages which are typical using other kinds of coagulation instruments.

US Patent Publication 2017/0193158 of Heindl, et al., describes a data processing method performed by a computer for supporting a medical brain mapping procedure, comprising the steps of receiving a microscope image of a patient's cortex and superimposing stimulation response marks onto the microscope image, wherein a stimulation response mark indicates the patient's response to an electrical stimulation of the cortex by a stimulation probe at a position associated with the stimulation response mark.

US Patent 6,240,308 to Hardy, et al., describes a method and apparatus for archiving and simultaneous display of brain scan images and a plurality of brain maps. The brain maps are proportioned to the individual brain of the scan images by a three-dimensional alignment process. Two-dimensional and three-dimensional displays are supported.

US Patent Publication 2014/0200874 of Zeng, et al., describes a map generator that can be programmed to generate a multi-parameter graphical map by encoding at least two different physiological parameters for a geometric surface, corresponding to tissue of a patient, using different color components of a multidimensional color model such that each of the different physiological parameters is encoded by at least one of the different color components.

US Patent Publication 2003/0065243 of Tanner describes a method for determining the function of a particular area of the brain, wherein at least one point on the brain is stimulated and/or inhibited, and from the presence of a perceived but not actually present sensory impression as a result of a stimulation pattern and/or a not perceived but actually present sensory impression as a result of an inhibiting pattern, the stimulated or inhibited area of the brain is functionally assigned, and to a device for determining the function of a particular area of the brain, comprising at least one device for stimulating and/or inhibiting at least one particular area of a brain and a device for generating a visual and/or acoustic and/or sensory and/or olfactory sensory impression.

### SUMMARY

There is provided in accordance with an embodiment of the present disclosure, a brain signal tracking system, including a probe configured to be inserted into, and moved around in, blood vessels of a brain of a living subject, and including a probe electrode, a signal generator configured to generate a calibration signal to be emitted by the probe electrode at different locations in the blood vessels, a tracking subsystem configured to track the different locations of the electrode in the blood vessels, head surface electrodes configured to be applied to a head of the living subject, respective ones of the head surface electrodes being configured to detect the calibration signal at respective amplitude values, with a distribution of the respective amplitude values over the respective head surface electrodes being indicative of a corresponding one of the tracked locations from which the calibration signal was emitted by the probe electrode, and processing circuitry configured to find a function to compute a source location in the brain of a brain signal from a distribution of respective amplitude values of the brain signal over the respective head surface electrodes responsively to the distribution of the respective amplitude values of the calibration signal over the respective head surface electrodes for ones of the tracked locations of the probe electrode.

Further in accordance with an embodiment of the present disclosure after the probe has been removed from the brain, the head surface electrodes are configured to detect a distribution of respective amplitude values of a first brain signal over the respective head surface electrodes, and the processing circuitry is configured to compute the source location in the brain of the first brain signal from the function using the distribution of the respective amplitude values of the first brain signal over the respective head surface electrodes as input to the function.

Still further in accordance with an embodiment of the present disclosure the calibration signal is a pulsed signal.

Additionally, in accordance with an embodiment of the present disclosure the tracking subsystem includes a location tracking sensor included in the probe.

Moreover, in accordance with an embodiment of the present disclosure the tracking subsystem includes a location pad having at least one magnetic field radiator configured to transmit alternating magnetic fields into a region where the brain is located, the location tracking sensor including a coil to detect at least part of the transmitted alternating magnetic fields.

There is also provided in accordance with another embodiment of the present disclosure, a brain signal tracking method, including generating a calibration signal to be emitted at different locations in blood vessels of a brain of a living subject by a probe electrode of a probe configured to be inserted into, and moved around in, the blood vessels, tracking the different locations of the probe electrode in the blood vessels, detecting the calibration signal at respective amplitude values by respective head surface electrodes configured to be applied to a head of the living subject, with a distribution of the respective amplitude values over the respective head surface electrodes being indicative of a corresponding one of the tracked locations from which the calibration signal was emitted by the probe electrode, and finding a function to compute a source location in the brain of a brain signal from a distribution of respective amplitude values of the brain signal over the respective head surface electrodes responsively to the distribution of the respective amplitude values of the calibration signal over the respective head surface electrodes for ones of the tracked locations of the probe electrode.

Further in accordance with an embodiment of the present disclosure, the method includes after the probe has been removed from the brain, detecting a distribution of respective amplitude values of a first brain signal over the respective head surface electrodes, and computing the source location in the brain of the first brain signal from the function using the distribution of the respective amplitude values of the first brain signal over the respective head surface electrodes as input to the function.

Still further in accordance with an embodiment of the present disclosure the calibration signal is a pulsed signal.

Additionally, in accordance with an embodiment of the present disclosure, the method includes transmitting alternating magnetic fields into a region where the brain is located, and detecting at least part of the transmitted alternating magnetic fields by a location tracking sensor included in the probe.

### BRIEF DESCRIPTION OF THE DRAWINGS

The present invention will be understood from the following detailed description, taken in conjunction with the drawings in which:
Figs. 1A and 1B are schematic, pictorial illustrations of probe-based cerebrovascular position tracking systems, in accordance with embodiments of the present invention;
Fig. 2A is a schematic view of a guidewire for use in the systems of Fig. 1A and 1B;
Fig. 2B is a cross-sectional view of the guidewire through line B:B of Fig. 2A;
Fig. 2C is a schematic view of a flexible tube for use in the systems of Fig. 1A and 1B;
Fig. 2D is a cross-sectional view of the flexible tube through line D:D of Fig. 2C;
Fig. 2E is a cross-section view of the flexible tube of Fig. 2D being slid over the guidewire of Fig. 2B;
Fig. 3 is a flowchart showing exemplary steps in a first method of operation of the systems of Figs 1A and 1B;
Fig. 4 is a schematic view of an alternative flexible probe for use with the systems of Figs. 1A and 1B;
Fig. 5A is a schematic view showing placement of head surface electrodes on a head of a living subject for use with the systems of Figs. 1A and 1B;
Fig. 5B is a schematic cutaway view showing a flexible probe inside blood vessels in the brain of the living subject of Fig. 5A; and
Figs. 6A and 6B are two parts of a flowchart of a second method of operation of the systems of Figs 1A and 1B.

### DESCRIPTION OF EXAMPLE EMBODIMENTS

### OVERVIEW

EEG (electroencephalograph) signals may be measured by applying electrodes to a patient's head. EEG signals may be used to give a first approximation to locations originating brain activity, but deriving these locations is difficult computationally, and is also relatively inexact.

Additionally, there are many conditions, such as Parkinson's disease, tinnitus, deep depression, sudden seizures, which may be caused by some defect in the functioning of the brain. Present systems use external electrodes attached to the patient's head to acquire EEG signals, but these signals do not provide sufficient detail of brain functioning.

In embodiments of the present invention a source location of a brain signal may be more accurately computed using head surface electrodes based on using a flexible probe to calibrate the head surface electrodes.

In particular, the flexible probe is inserted into, and moved around in, blood vessels of the brain of the living subject and an electrode of the flexible probe is tracked using a tracking subsystem. In some embodiments, the probe includes a location tracking sensor, which may include a coil. At each tracked location, a calibration signal is emitted by the probe electrode for detection by the head surface electrodes. In some embodiments, the calibration signal is a pulsed signal, optionally pulsed at known intervals, for easier recognition.

For any one tracked location of the probe electrode, the calibration signal is generally detected by respective ones of the head surface electrodes at respective amplitude values resulting in a distribution of respective amplitude values over the respective head surface electrodes. The distribution is indicative of the corresponding tracked location of the probe electrode from which the calibration signal was emitted. The respective distributions for respective tracked locations may be used as a calibration data set for calibrating the head surface electrodes.

The calibration data set is analyzed to find a function from which to compute a source location in the brain of a brain signal from a distribution of respective amplitude values of the brain signal over the respective head surface electrodes. The function may be found using machine learning techniques, by way of example only. In some embodiments, a source location in the brain of a brain signal may be computed from a distribution of respective amplitude values of the brain signal over the respective head surface electrodes by interpolating over the configuration data set. In these embodiments, the function comprises the configuration data set.

Therefore, after the flexible probe has been removed from the brain, the head surface electrodes may detect a distribution of respective amplitude values of another brain signal over the respective head surface electrodes. The function may then be used (e.g., by interpolating over the configuration data set of the function) to compute the source location in the brain of this other brain signal from the function using the distribution of the respective amplitude values of this other brain signal over the respective head surface electrodes as input to the function.

### SYSTEM DESCRIPTION

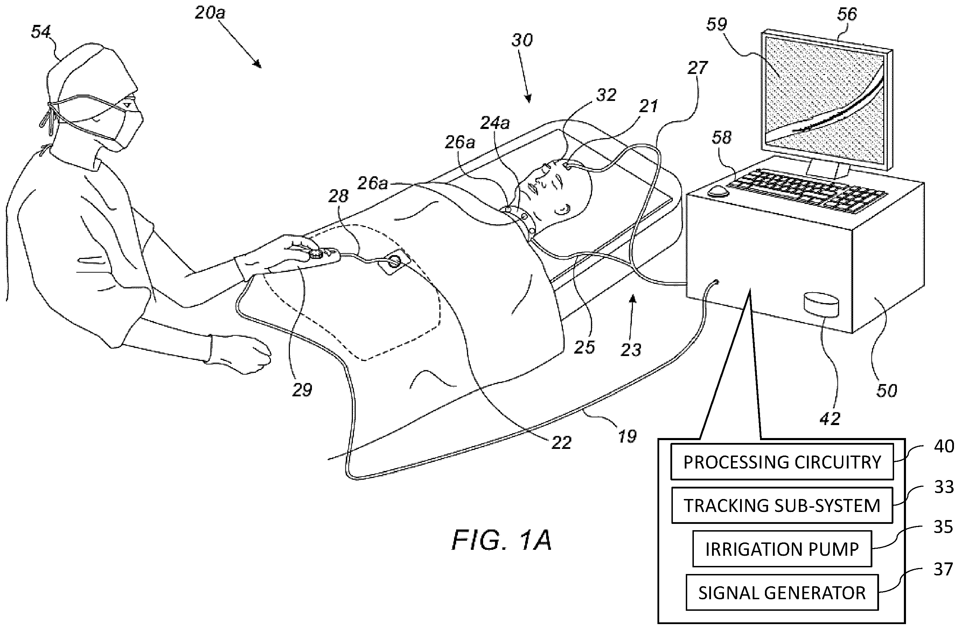

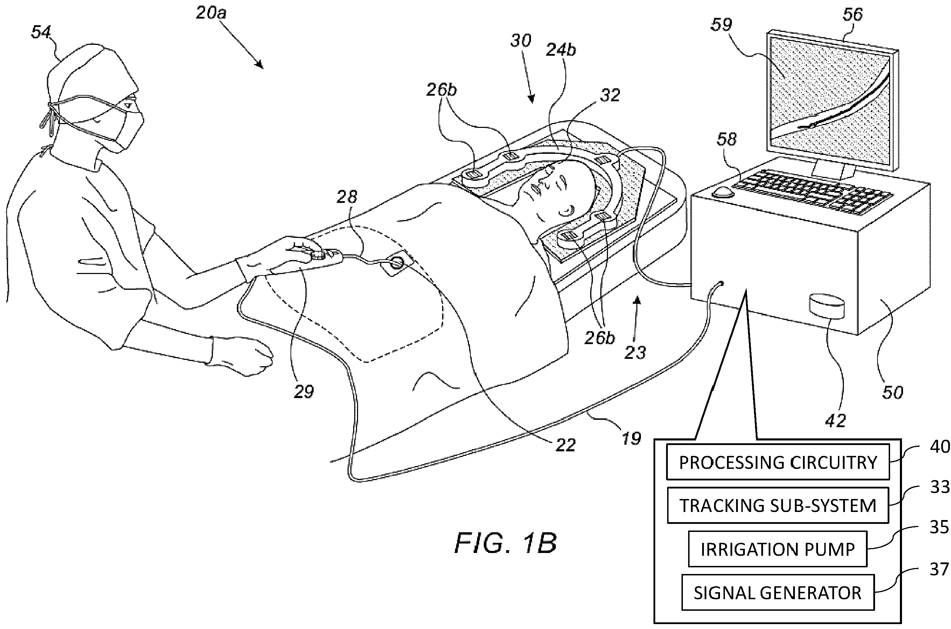

Figs. 1A and 1B are schematic, pictorial illustrations of probe-based cerebrovascular position tracking systems 20a and 20b, in accordance with embodiments of the present invention.

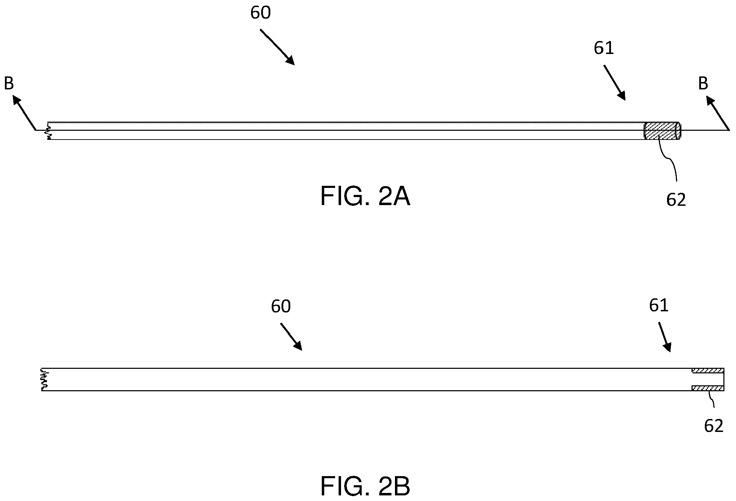

In some embodiments, prior to performing the procedure, CT images of a patient 32 are acquired. The CT images are stored in a memory 42 for subsequent retrieval by processing circuitry 40. The processing circuitry 40 uses the images to present, for example, a brain section image 59 on a display 56. During the disclosed procedure, tracking systems 20a and 20b register a position of a distal end of a guidewire (shown in Figs. 2A-B) and/or a flexible tube 28 inside the patient's brain, with frames of reference of brain images of the patient 32, herein assumed by way of example to comprise real-time fluoroscopic images.

The position of the distal end of the flexible tube 28 and/or the guidewire may be tracked using a magnetic tracking subsystem 23, which tracks position and orientation coordinates of a magnetic sensor fitted at the distal ends. The magnetic tracking subsystem 23 may form part of a tracking subsystem 33. In some embodiments, the flexible tube 28 and/or the guidewire may be tracked using any suitable tracking system for example, but not limited to, an electrical based tracking system using multiple head surface electrodes (not shown) to track the position of the flexible tube 28 and/or the guidewire based on a signal emitted by an electrode of the flexible tube 28 and/or the guidewire.

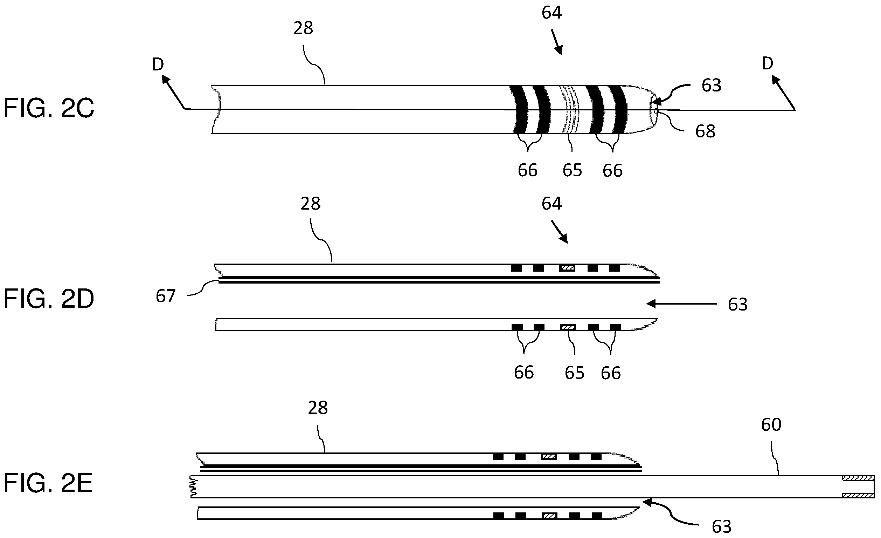

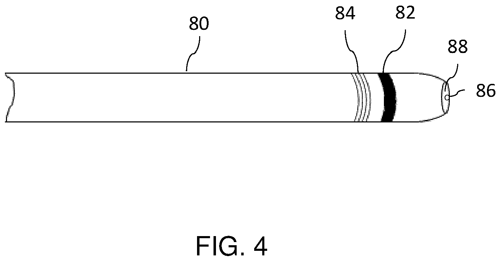

Using magnetic position tracking subsystem 23, a physician 54 advances the distal end of the guidewire and/or the flexible tube 28 through blood vessels, usually arteries, to perform an invasive therapeutic procedure and/or to map electrical activity of the brain (described in more detail with reference to Figs. 2A E, and 3) or to calibrate head surface electrodes (described in more detail with reference to Figs. 4-6). In some embodiments, the guidewire (described in more detail with reference to Figs. 2A, B and E) is first inserted into the body and guided to the blood vessels of the brain. A lumen of the flexible tube 28 is then slid over the guidewire until the flexible tube 28 is in place. The guidewire is then optionally removed.

In system 20a, shown in Fig. 1A, a location pad 24a, comprised in magnetic tracking subsystem 23, is implemented as a collar around the neck of patient 32. By putting location pad 24a around the neck, location pad 24a is configured to automatically compensate for patient head movement. Location pad 24a comprises magnetic field radiators 26a which are fixed in position relative to the head of patient 32 and which transmit alternating sinusoidal magnetic fields into a region 30 where the head of patient 32 is located. A console 50 electrically drives radiators 26a via a cable 25. In an embodiment, further compensation of head motion is provided by attaching a reference sensor 21 to the patient's forehead. Console 50 is configured to receive signals from reference sensor 21 via a cable 27. A location tracking system that comprises a neck collar location pad is described in U.S. Patent Application 16/248,393, filed January 15, 2019, entitled "Position Sensor on Brain Clot Sheath and Location Pad Collar," which is assigned to the assignee of the present patent application. The alternating sinusoidal magnetic fields are detected by the magnetic sensor(s) in the guidewire and/or the flexible tube 28. The transmitted alternating magnetic fields generate signals in the magnetic sensors, which are indicative of position and/or direction. The sensor(s) generates position signals which are sent via a cable 19 for processing via the tracking subsystem 33.

Physician 54, operating system 20a, holds a controller handle 29, which is connected to the proximal end of flexible tube 28 and/or the guidewire. Controller handle 29 allows the physician to advance and navigate flexible tube 28 and/or the guidewire in the brain, for example, through an entry point 22 at an artery at a thigh of patient 32. As noted above and described below, physician 54 navigates the distal end of the guidewire and/or flexible tube 28 using position and orientation signals from a magnetic sensor fitted at the distal end of the guidewire and/or flexible tube 28. Console 50 receives the position signals through the cable 19 that connects to flexible tube 28 via handle 29.

Elements of system 20a, including radiators 26a, are controlled by processing circuitry 40, comprising a processing unit communicating with one or more memories (e.g., the memory 42). Processing circuitry 40 may be mounted in console 50, which comprises operating controls 58 that typically include a keypad and/or a pointing device such as a mouse or trackball. Physician 54 uses operating controls on handle 29 to interact with the processing circuitry 40 while performing the registration of system 20a. During the registration process, the brain section image 59 is presented on display 56. Subsequent to the registration process described above, physician 54 uses the operating controls to advance the distal end of the guidewire and/or the flexible tube 28 to one or more desired locations in the brain.

Processing circuitry 40 uses software stored in the memory 42 to operate system 20a. In practice, some or all of the functions of the processing circuitry 40 may be combined in a single physical component or, alternatively, implemented using multiple physical components. These physical components may comprise hard-wired or programmable devices, or a combination of the two. In some embodiments, at least some of the functions of the processing circuitry 40 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

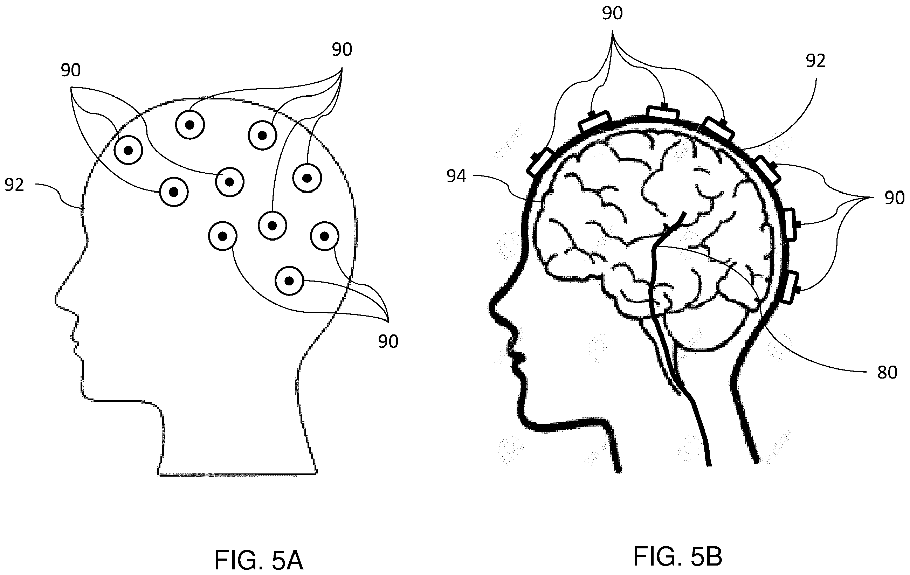

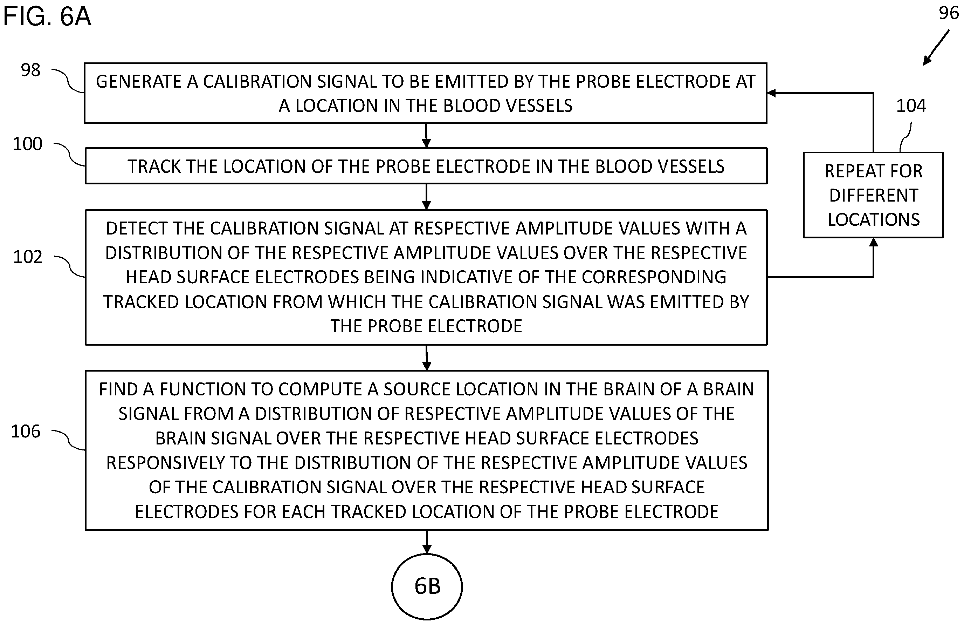

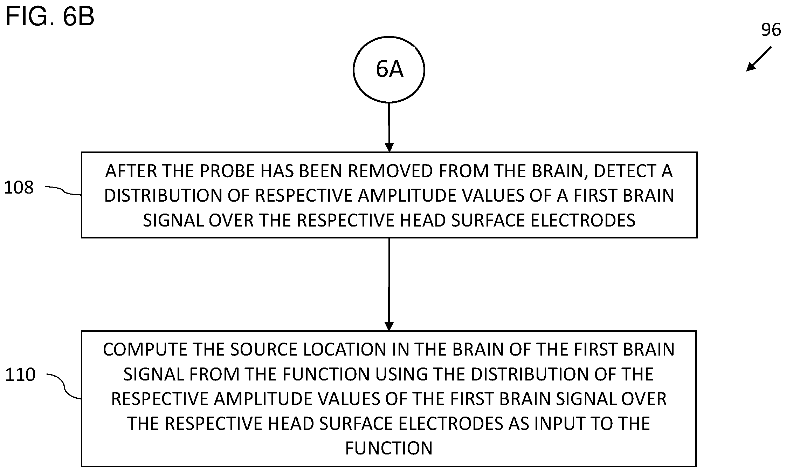

In some embodiments, the console 50 may also include an irrigation pump 35 for pumping irrigation fluid through a lumen of the flexible tube 28. In some embodiments, the console 50 may include a signal generator 37 described in more detail with reference to Figs. 5B and 6.

System 20b, shown in Fig. 1B, has a different magnetic location pad design, namely a location pad 24b. As seen, location pad 24b is fixed to the bed, and irradiators 26b surround a patient headrest horizontally. In this example, system 20b lacks reference sensor 21, and therefore the head of the patient must be harnessed to prevent motion of the head. Other components of system 20b are generally identical to those of system 20a. A location tracking system using a location pad similar to location pad 24b is described in U.S. Patent Publication 2019/0046272.

Systems 20a and 20b shown in Figs. 1A and 1B are chosen purely for the sake of conceptual clarity. Other system elements may be included, for example additional controls on handle 29 for controlling additional tooling such as for drug delivery.

Carto® magnetic tracking systems, which track a location and orientation of a magnetic position sensor in an organ of a body using techniques similar to those applied by systems 20a and 20b, are produced by Biosense-Webster (Irvine, California). In general, position sensing using current distribution measurements and/or external magnetic fields are described in detail in U.S. Patents 5,391,199, 6,690,963, 6,484,118, 6,239,724, 6,618,612, 6,332,089, 7,756,576, 7,869,865, and 7,848,787, in PCT Patent Publication WO 96/05768, and in U.S. Patent Application Publications 2002/0065455 A1, 2003/0120150 A1 and 2004/0068178 A1.

Reference is now made to Figs. 2A and 2B. Fig. 2A is a schematic view of a guidewire 60 for use in the systems 20a and 20b of Fig. 1A and 1B. Fig. 2B is a cross-sectional view of the guidewire 60 through line B:B of Fig. 2A. The guidewire 60 is configured to be inserted into blood vessels of a brain of a living subject. The guidewire includes a distal end 61. The tracking subsystem 33 (Figs. 1A and 1B) includes a location tracking sensor 62, which is comprised in the distal end 61 of the guidewire 60. The tracking subsystem 33 is configured to track a position of the distal end of the guidewire 60 from signals generated by the location tracking sensor 62 in response to the magnetic field radiators 26a or 26b (Figs. 1A and 1B).

The guidewire 60 may be formed from any suitable materials or mixture of materials, such as a metal spiral and/or a solid rod core-wire, which may include one or more surface channels cut into the surface of the solid rod core-wire for accepting electrical wires to connect to the location tracking sensor 62. In some embodiments, the surface channel(s) spirals around the core wire away from the distal end towards the proximal end of the guidewire 60. The solid rod core-wire may be formed from any suitable material for example, but not limited to, stainless steel, cobalt chromium or nitinol. The guidewire 60 may have any suitable length. In some embodiments, the guidewire 60 has a length in the range of 1-3 meters, for example about 2 meters. The guidewire 60 may have any suitable outer diameter. In some embodiments the guidewire 60 may have an outer diameter in the range of 200-900 microns, for example, 300 microns. The distal end of the guidewire 60 may be tapered or rounded, for example, by grinding the distal end.

A shrink-sleeve (not shown) may be disposed over the guidewire 60. The shrink-sleeve helps retain electrical wires in the surface channel. The shrink-sleeve may be formed from any suitable material, for example, a polyethylene terephthalate (PET) or a fluoropolymer. The shrink-sleeve typically covers the guidewire 60 for its entire length. In some embodiments, the shrink-sleeve may only partially cover the guidewire 60.

The location tracking sensor 62 may comprise a coil, for example, a wound coil. The coil may have any suitable diameter, for example, between 0.5 mm and 1 mm, and in some embodiments, 0.65 mm. The coil may include any suitable insulated wire. In some embodiments, the coil is wound from insulated copper wire around the distal end 61. The distal end 61 may have a slightly smaller outer diameter than the rest of the guidewire 60 in order that the wound coil is about level with the rest of the surface of the guidewire 60. The gauge of the wire may be any suitable gauge. In some embodiments, the wire is 60-gauge insulated copper wire, which has an outer diameter of about 8 microns. The coil is covered with any suitable cover, for example, but not limited to, the shrink sleeve described above, a plastic cover such as a plastic tube, or with a coating such an as enamel or epoxy paint, shrink sleeve, or metal cover. A metal cover may also provide shielding from high frequency electromagnetic interference.

Reference is now made to Figs. 2C and 2D. Fig. 2C is a schematic view of the flexible tube 28 for use in the systems 20a and 20b of Fig. 1A and 1B. Fig. 2D is a cross-sectional view of the flexible tube 28 through line D:D of Fig. 2C.

The flexible tube 28 includes a lumen 63 extending from a proximal end to a distal end 64 of the flexible tube 28. The lumen 63 is sized to fit around the guidewire 60 (as shown in Fig. 2E). The flexible tube 28 is configured to be inserted into, and moved around in, blood vessels of the brain of the living subject. The flexible tube 28 is generally formed as an elongated tube with a tapered distal end providing a smooth surface without any sharp corners or edges. In some embodiments, at least a distal portion of at least 5 cm in length of the flexible tube 28 has a maximum outer diameter of 3 mm, e.g., 1 mm. The length of the distal portion having a maximum outer diameter of 3 mm may be greater than 5 cm, for example, up to 20 cm, or for a majority, or for all of the length, of the flexible tube 28. In some embodiments, the flexible tube 28 is magnetic resonance imaging (MRI) compatible. The flexible tube 28 may be formed from polyurethane, a thermoplastic elastomer such as polyether block amide (PEBA) for example, PEBAX® or VESTAMID®, and/or silicon with biocompatible coating.

The tracking subsystem 33 (Figs. 1A and 1B) includes a location tracking sensor 65 comprised in the distal end of the flexible tube 28. In Figs. 2C and D, the location tracking sensor 65 is disposed between two sets of bipolar electrode pairs 66. The location tracking sensor 65 may be disposed in any suitable location. The tracking subsystem 33 is configured to track the locations of the bipolar electrode pair(s) 66 in the blood vessels. As described above with reference to Figs. 1A and 1B, the tracking subsystem 33 includes the location pad 24a or 24b having the magnetic field radiator(s) 26a or 26b configured to transmit alternating magnetic fields into the region 30 where the brain is located. In some embodiments, the location tracking sensor 65 includes a coil to detect at least part of the transmitted alternating magnetic fields. Fig. 2D shows the coil as being wound close to the surface of the flexible tube 28. The flexible tube 28 includes a groove therein in which the location tracking sensor 65 is disposed (e.g., wound in the groove).

In some embodiments, the coil is disposed beneath an outer casing of the flexible tube 28 and is generally disposed coaxially with the flexible tube 28. The coil may have any suitable diameter. The coil may include any suitable insulated wire. In some embodiments, the coil is wound from insulated copper wire. The gauge of the wire may be any suitable gauge. In some embodiments, the wire is 60-gauge insulated copper wire, which has an outer diameter of about 8 microns. The coil is covered with any suitable cover, for example, but not limited to, a shrink sleeve, a plastic cover such as a plastic tube, with a coating such an as enamel or epoxy paint, or a metal cover. A metal cover may also provide shielding from high frequency electromagnetic interference.

The bipolar electrode pair(s) 66 are disposed in the distal end 64 of the flexible tube 28. The bipolar electrode pair(s) 66 are configured to detect signals indicative of respective electrical activity of the brain at respective locations in the blood vessels of the brain. The flexible tube 28 may include at least one groove therein in which the bipolar electrode pair(s) 66 are disposed. Each electrode may be crimped or otherwise attached to the flexible tube 28. The flexible tube 28 may have any suitable number of bipolar electrode pairs 66. The bipolar electrode pairs 66 may detect signals indicative of electrical activity in the brain without having to use body/head surface electrodes for detecting the electrical activity. In some embodiments, body/head surface electrodes may be used as part of the tracking subsystem 33 to track a location of the bipolar electrode pair(s) 66 of the flexible tube 28. The bipolar electrode pair(s) 66 may be composed of any suitable material for example, but not limited to, platinum-iridium alloy. The electrodes may have any suitable width, for example, in a range of 0.2 mm to 0.6 mm, and typically 0.4 mm.

An optical fiber 67 and a lens 68 may be connected to the flexible tube 28 to capture images in the brain. In other embodiments, the optical fiber 67 and lens 68 are optional. The optical fiber 67 and wires connected to the location tracking sensor 65 and the bipolar electrode pair(s) 66 may be fed down the lumen 63 and attached to the wall of the lumen 63 using any suitable adhesive.

The irrigation pump 35 (Fig. 1) may be configured to pump irrigation fluid into the brain via the lumen 63 (or another channel disposed in the lumen 63) of the flexible tube 28. The irrigation fluid may be any suitable fluid, for example, but not limited to, a saline. The irrigation rate may be any suitable irrigation rate, for example, but not limited to, in the range of 5 to 25 ml/min., and typically 15ml/min. The irrigation fluid may provide lubrication for moving the flexible tube 28 as well as preventing blood clots. In other embodiments, the irrigation pump 35 is optional.

Reference is now made to Fig. 2E, which is a cross-section view of the flexible tube 28 of Fig. 2D being slid over the guidewire 60 of Fig. 2B. The lumen 63 of the flexible tube 28 is configured to slide axially over the guidewire 60 so that a length of the lumen surrounds a length of the guidewire in the blood vessels of the brain. In this manner, after the guidewire 60 is positioned correctly in the blood vessels of the brain, the lumen 63 of the flexible tube 28 is slid over the guidewire 60 until the flexible tube 28 is in place. Once the flexible tube 28 is in place, the guidewire 60 may be optionally removed. The flexible tube 28 is generally very flexible so that the flexible tube 28 cannot be deflected or torqued by the physician 54. Once in the blood vessels of the brain, the flexible tube 28 may be moved longitudinally within the blood vessels by the physician 54.

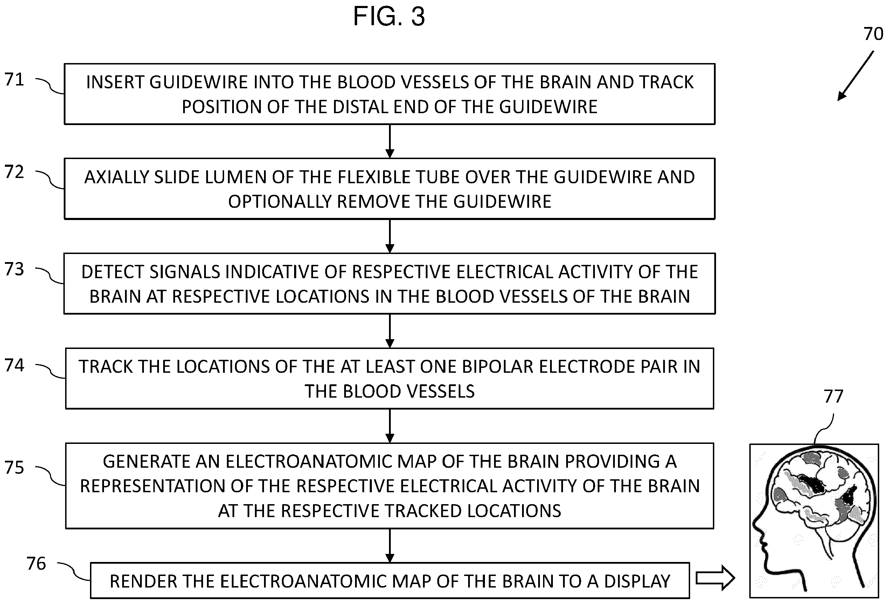

Reference is now made to Fig. 3, which is a flowchart 70 showing exemplary steps in a first method of operation of the systems 20a and 20b of Figs 1A and 1B. The physician 54 (Fig. 1) inserts (block 71) the guidewire 60 (Fig. 2A) into the blood vessels of the brain. The tracking subsystem 33 is configured to track the position of the distal end 61 of the guidewire 60. The tracking subsystem 33 may include any suitable tracking method for example, using magnetic tracking with the location tracking sensor 62 of the guidewire 60, or using an imaging method such as real-time fluoroscopy. The physician 54 axially slides (block 72) the lumen 63 of the flexible tube 28 (Figs. 2C-E) over the guidewire 60. Once the flexible tube 28 is in place, the physician 54 optionally removes the guidewire 60 while leaving the flexible tube 28 in place in the blood vessels of the brain.

The bipolar electrode pairs 66 may then be moved by moving the distal end 64 of the flexible tube 28 around in the blood vessels of the brain. The bipolar electrode pair(s) 66 are configured to detect (block 73) signals indicative of respective electrical activity of the brain at respective locations in the blood vessels of the brain. The electrical activity of the brain at each of the respective locations is generally detected without using electrodes applied outside of the blood vessels (e.g., on the surface of the scalp). The tracking subsystem 33 (Figs. 1A and 1B), which comprises the location tracking sensor 65 of the flexible tube 28, is configured to track (block 74) the locations of the bipolar electrode pair(s) 66 in the blood vessels. The processing circuitry 40 (Figs. 1A and 1B) is configured to generate (block 75) an electroanatomic map 76 of the brain providing a representation of the respective electrical activity of the brain at the respective tracked locations. In some embodiments, the electroanatomic map 76 is a volumetric map of the respective electrical activity of the brain at the respective locations. The processing circuitry 40 is configured to render (block 76) the electroanatomic map 77 to the display 56 (Figs. 1A and 1B).

While the brain is not a cyclic system like the heart, the generated electroanatomic map 77 allows tracing, in time and space, of firing instances of the brain, illustrating for example firing during a seizure. The firing sequences may or may not be repeatable. In generating the electroanatomic map 77, the detected electrical activity may be processed to provide data other than firing instances, for example, but not limited to, conduction paths, bursts, irregularities, and/or underlying background activity. The various features of the electroanatomic map 77 may be illustrated using any one or more of the following: colors, shading, arrows, lines, and/or annotations. The electroanatomic map 77 may show areas of higher amplitude and/or more frequent electrical activity using a certain presentation scheme (e.g., thicker lines or arrows, darker colors or shading) and areas of lower amplitude and/or less frequent electrical activity using a certain presentation scheme (e.g., thinner lines or arrow, lighter colors or shading).

The electroanatomic map 77 may allow drilling down to different sections of the electroanatomic map 77 and/or viewing different slices of the electroanatomic map 77. In some embodiments, the electroanatomic map 77 may be represented as a shell (e.g. a type of skeletal structure or translucent map) so that inner features of the map 77 may also be visible from the outside of the brain.

The electroanatomic map 77 may be particularly useful, for example, in investigating patients with recurring problems, such as those with chronic pain, tinnitus, and/or seizures, where extra activity of certain areas of the brain is suspected. Areas of the brain which, on a relative basis, are more active can be further investigated at a higher resolution, and the map of such areas may be helpful in treating the patient. In some embodiments, the mapping is performed during an episode where abnormal brain activity is suspected, for example, during a seizure, during a spell of depression, or in response to a trigger which is suspected to produce some detectable brain activity.

The description below, with reference to Figs. 4-6, describes calibrating head surface electrodes to provide more accurate locations for sources of electrical activity within a brain.

Reference is now made to Fig. 4 which is a schematic view of a flexible probe 80 for use with the systems 20a and 20b of Figs. 1A and 1B.

The probe 80 is substantially the same as the flexible tube 28 of Figs. 2C and 2D except that the bipolar electrode pair(s) 66 are replaced with a single unipolar probe electrode 82. In some embodiments, the flexible tube 28 may be used instead of the flexible probe 80, while configuring one electrode among the bipolar electrode pair(s) 66 to act as the unipolar probe electrode 82. In other embodiments, any suitable probe may be inserted into the blood vessels of the brain to perform the method described with reference to Figs. 5 and 6.

In common with the flexible tube 28, the distal end of the flexible probe 80 is generally dimensioned to fit into blood vessels of the brain. In some embodiments, the flexible probe 80 has a diameter of 3 mm or less, for example, 1mm. In some embodiments, the flexible probe 80 is magnetic resonance imaging (MRI) compatible. The flexible probe 80 also includes a location tracking sensor 84, which is part of the tracking subsystem 33 (Figs. 1A and 1B). The location tracking sensor 84 is disposed adjacent to the probe electrode 82 in order to keep a more accurate track on the location of the probe electrode 82. The tracking subsystem 33 is configured to track the locations of the probe electrode 82 in the blood vessels. As described above with reference to Figs. 1A and 1B, the tracking subsystem 33 includes the location pad 24a or 24b having the magnetic field radiator(s) 26a or 26b configured to transmit alternating magnetic fields into the region 30 where the brain is located. In some embodiments, the location tracking sensor 84 includes a coil (substantially the same as the coil described with reference to Figs. 2C-D) to detect at least part of the transmitted alternating magnetic fields.

The flexible probe 80 may also include an optical fiber and lens 86 and at least one irrigation channel or lumen 88 therein. The optical fiber and lens 86 and the irrigation channel(s) 88 are substantially the same as the optical fiber 67, lens 68, and the lumen 63, respectively, described above with reference to Figs. 2C and 2D. In other embodiments, the optical fiber and lens 86 and/or the irrigation channel(s) 88 are optional.

Reference is now made to Fig. 5A, which is a schematic view showing placement of head surface electrodes 90 on a head 92 of a living subject for use with the systems 20a and 20b of Figs. 1A and 1B.

The head surface electrodes 90 are configured to be applied to the head 92 of the living subject, typically on all sides of the scalp and even below the scalp in certain areas (e.g., at the back of the head 92), in any suitable configuration. A higher density placement of the head surface electrodes 90 on the head 92 may lead to more accurate location results but may lead to more computation to compute the location of electrical activity with the brain of the living subject. The head surface electrodes 90 are typically connected by leads (not shown) to the console 50 of Figs. 1A and 1B.

Reference is now made to Fig. 5B, which is a schematic cutaway view showing the flexible probe 80 inside blood vessels in a brain 94 of the living subject of Fig. 5A. The flexible probe 80 is moved from location to location in the brain to calibrate the head surface electrodes 90. At any one location, a calibration signal, typically a pulsed signal, is generated by the signal generator 37 (Figs. 1A and 1B) and emitted by the probe electrode 82 (Fig. 4) for detection by the head surface electrodes 90. The location of the probe electrode 82 is also tracked using the location tracking sensor 84 and the tracking subsystem 33 (Fig. 1). Due to the different distances from the tracked location of the probe electrode 82 to the head surface electrodes 90 and the different electrical properties of the tissue between the tracked location of the probe electrode 82 and the respective head surface electrodes 90, the distribution or spread of amplitude values of the calibration signal detected by the head surface electrodes 90 is indicative of the tracked location. The probe electrode 82 is moved to several locations in the blood vessels of the brain 94, generating a calibration data set comprising the distribution of amplitude values of the calibration signal over the head surface electrodes 90 at each of the tracked locations. The calibration data set is then processed to yield a function to allow computation of a source location of a brain signal, even after the flexible probe 80 has been removed from the brain 94, according to the distribution of amplitude values of that brain signal over the head surface electrodes 90.

Reference is now made to Figs. 6A and 6B, which are two parts of a flowchart 96 of a second method of operation of the systems 20a and 20b of Figs 1A and 1B. Reference is first made to Fig. 6A.

The signal generator 37 (Figs. 1A and 1B) is configured to generate (block 98) a calibration signal to be emitted by the probe electrode 82 (Fig. 4) at a location in the blood vessels of the brain 94 (Fig. 5B). In some embodiments, the calibration signal is a pulsed signal, typically pulsed at known intervals. The tracking subsystem 33 (Figs. 1A and 1B) is configured to track (block 100) the location of the probe electrode 82 in the blood vessels.

Respective ones of the head surface electrodes 90 (Figs. 5A and 5B) are configured to detect (block 102) the calibration signal at respective amplitude values, with a distribution of the respective amplitude values over the respective head surface electrodes 90 being indicative of the corresponding tracked location from which the calibration signal was emitted by the probe electrode 92.

The steps of blocks 98-102 are repeated (block 104) for different locations. The accuracy of the calibration may be increased with the number of different locations. The probe electrode 82 is moved to several locations in the blood vessels of the brain 94, generating a calibration data set comprising the distribution of amplitude values of the calibration signal over the head surface electrodes 90 at each of the tracked locations. The processing circuitry 40 compiles and stores the calibration data set in the memory 42 (Figs. 1A and 1B).

The processing circuitry 40 (Figs. 1A and 1B) is configured to find (block 106) a function to compute a source location in the brain 94 of a brain signal from a distribution of respective amplitude values of the brain signal over the respective head surface electrodes 90 responsively to the calibration data set which comprises the distribution of the respective amplitude values of the calibration signal over the respective head surface electrodes 90 for each of the tracked locations of the probe electrode 82 (Fig. 4). The function may be found using machine learning techniques, by way of example only. In some embodiments, a source location in the brain of a brain signal may be computed from a distribution of respective amplitude values of the brain signal over the respective head surface electrodes by interpolating over the configuration data set. In these embodiments, the function comprises the configuration data set and the step of block 106 includes defining the function to include the configuration data set.

Reference is now made to Fig. 6B.

After the flexible probe 80 (Fig. 4) has been removed from the brain 94, the head surface electrodes 90 are configured to detect (block 108) a distribution of respective amplitude values of a first brain signal over the respective head surface electrodes 90. As there may be a variety of brain electrical activity occurring at the same time from different locations in the brain, the signals detected by the head surface electrodes 90 may be filtered to remove signals below a given amplitude, and/or below or above a given frequency in order to isolate the brain activity of interest prior to performing the step of block 110, described below. For example, brain activity from a seizure may be above a given amplitude. Other activity below this amplitude may then be filtered prior to performing the step of block 110.

The processing circuitry is configured to compute (block 110) the source location in the brain 94 of the first brain signal from the function using the distribution of the respective amplitude values of the first brain signal over the respective head surface electrodes 90 as input to the function (e.g., by interpolating over the configuration data set of the function based on the distribution of the respective amplitude values of the first brain signal over the respective head surface electrodes 90).

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

## Claims

1. A brain signal tracking system, comprising:
a probe configured to be inserted into, and moved around in, blood vessels of a brain of a living subject, and including a probe electrode;
a signal generator configured to generate a calibration signal to be emitted by the probe electrode at different locations in the blood vessels;
a tracking subsystem configured to track the different locations of the electrode in the blood vessels;
head surface electrodes configured to be applied to a head of the living subject, respective ones of the head surface electrodes being configured to detect the calibration signal at respective amplitude values, with a distribution of the respective amplitude values over the respective head surface electrodes being indicative of a corresponding one of the tracked locations from which the calibration signal was emitted by the probe electrode; and
processing circuitry configured to find a function to compute a source location in the brain of a brain signal from a distribution of respective amplitude values of the brain signal over the respective head surface electrodes responsively to the distribution of the respective amplitude values of the calibration signal over the respective head surface electrodes for ones of the tracked locations of the probe electrode.

2. The system according to claim 1, wherein:
after the probe has been removed from the brain, the head surface electrodes are configured to detect a distribution of respective amplitude values of a first brain signal over the respective head surface electrodes; and
the processing circuitry is configured to compute the source location in the brain of the first brain signal from the function using the distribution of the respective amplitude values of the first brain signal over the respective head surface electrodes as input to the function.

3. The system according to claim 1, wherein the calibration signal is a pulsed signal.

4. The system according to claim 1, wherein the tracking subsystem includes a location tracking sensor comprised in the probe.

5. The system according to claim 4, wherein the tracking subsystem includes a location pad having at least one magnetic field radiator configured to transmit alternating magnetic fields into a region where the brain is located, the location tracking sensor including a coil to detect at least part of the transmitted alternating magnetic fields.

6. A brain signal tracking method, comprising:
generating a calibration signal to be emitted at different locations in blood vessels of a brain of a living subject by a probe electrode of a probe configured to be inserted into, and moved around in, the blood vessels;
tracking the different locations of the probe electrode in the blood vessels;
detecting the calibration signal at respective amplitude values by respective head surface electrodes configured to be applied to a head of the living subject, with a distribution of the respective amplitude values over the respective head surface electrodes being indicative of a corresponding one of the tracked locations from which the calibration signal was emitted by the probe electrode; and
finding a function to compute a source location in the brain of a brain signal from a distribution of respective amplitude values of the brain signal over the respective head surface electrodes responsively to the distribution of the respective amplitude values of the calibration signal over the respective head surface electrodes for ones of the tracked locations of the probe electrode.

7. The method according to claim 6, further comprising:
after the probe has been removed from the brain, detecting a distribution of respective amplitude values of a first brain signal over the respective head surface electrodes; and
computing the source location in the brain of the first brain signal from the function using the distribution of the respective amplitude values of the first brain signal over the respective head surface electrodes as input to the function.

8. The method according to claim 6, wherein the calibration signal is a pulsed signal.

9. The method according to claim 6, further comprising: transmitting alternating magnetic fields into a region where the brain is located; and detecting at least part of the transmitted alternating magnetic fields by a location tracking sensor comprised in the probe.
